# EUROPEAN PATENT APPLICATION

(11) **EP 1 952 835 A1**
(43) Date of publication of application: **06.08.2008**
(21) Application number: 08250351.7
(22) Date of filing: 29.01.2008
(51) Int. Cl.: A61M 5/142

(54) **Water-proof infusion pump keypad assembly and method for making the same**

(30) Priority: 30.01.2007 US 699828
(71) Applicant: Animas Corporation, West Chester PA 19380 (US)
(72) Inventor: Kiersh, Jeff, Exton, Pennsylvania 19341 (US)
(74) Representative: Tunstall, Christopher Stephen

(57) **Abstract**

An infusion pump and method of its manufacture according to the present invention are provided. The infusion pump has a pump casing made from a first material and defining a keypad assembly area. Disposed within the keypad assembly area and electrically coupled to the infusion pump is a flexible circuit. The keypad assembly is hermetically sealed over the flexible circuit to the keypad assembly area. The keypad assembly includes a frame made from a second material and defining a perimeter sized to mate with the keypad assembly area, the frame having an adhesive surface and an overmold surface, and a keypad made from a third material and in direct contact with the overmold surface of the frame.

## Description

### FIELD OF THE INVENTION

The present invention is directed to infusion pumps, and more particularly, to infusion pumps having a silicone keypad overmolded to a frame, which frame is chemically bonded to the pump casing forming a hermetic seal between the keypad assembly and the pump casing.

### BACKGROUND OF THE INVENTION

Generally, medical patients sometimes require precise delivery of either continuous medication or medication at set periodic intervals. Medical infusion pumps have been developed to provide controlled drug infusion wherein the drug can be administered at a precise rate that keeps the drug concentration within a therapeutic margin. Basically, the medical infusion pumps provide appropriate drug delivery to the patient at a controllable rate which does not require frequent attention.

Some infusion pumps are designed to be controlled by the patient through a user interface. These patient-controlled, infusion-on-demand pumps allow the user to manipulate some of the settings of the pump, but are also typically preprogrammed with limits. The user manipulated parameters are typically controlled by a pressure pad containing buttons that can be activated by the patient.

A common commercial use of the patient-controlled pumps are insulin pumps. An insulin pump is a medical device used for administering insulin in the treatment of diabetes mellitus. An insulin pump is an alternative to multiple daily injections of insulin by syringe or an insulin pen and allows for intensive insulin therapy when used in conjunction with blood glucose monitoring and carb counting.

Because insulin pumps travel with its user during their normal daily routine, the pumps, and their user interfaces, are exposed to environmental hazards and insults, such as external moisture and shock. Accordingly, there is a need for an insulin pump having a user interface that can withstand these insults.

### SUMMARY OF THE INVENTION

The present invention is directed to an infusion pump having a pump casing made from a first material, for example polycarbonate, and defining a keypad assembly area. Hermetically sealed to the keypad assembly area is a keypad assembly comprising a frame made from a second material and defining a perimeter sized to mate with the keypad assembly area. The frame has an adhesive surface and an overmold surface. Coupled to the overmold surface of the frame is a keypad made from a third material, preferably thermoplastic polyurethane elastomers TPU. According to an exemplary embodiment, a flexible circuit is disposed between the keypad assembly and the keypad assembly surface of the pump casing. The keypad is in direct contact with the overmold surface of the frame and the adhesive surface is chemically bonded to the keypad assembly area to hermetically seal the flexible circuit within the keypad assembly area of the infusion pump casing.

According to an exemplary embodiment, the method of manufacturing an infusion pump of the present invention includes providing a pump casing made from a first material and having a keypad assembly area. A frame comprising a second material and defining a perimeter sized to mate with the keypad assembly area is provided. The frame has an adhesive surface and an overmold surface. A keypad is then overmolded onto the overmold surface of the frame. According to an exemplary embodiment, a flexible circuit is placed within the keypad assembly area, and the adhering surface of the frame is chemically bonded to the keypad assembly area of the infusion pump casing to hermetically seal the flexible circuit within the infusion pump casing.

According to an exemplary embodiment, the method of assembling an infusion pump having a keypad assembly and a pump casing includes first cleaning the keypad assembly area with a solvent. According to an exemplary embodiment, a flexible circuit is placed within the keypad assembly area and electrically coupled to the pump. Next, adhesive is placed on the adhesive surface of the frame of the keypad assembly. The adhesive perimeter surface of the frame is aligned with the keypad assembly area, and the function keys are aligned with the electrical contact of the flexible circuit. The keypad assembly is placed over the keypad assembly area. When the adhesive cures, a hermetic seal is formed between the keypad assembly and the pump casing to seal the flexible circuit within the keypad assembly area of the pump casing.

### BRIEF DESCRIPTION OF THE DRAWINGS

The invention is best understood from the following detailed description when read in connection with the accompanying drawing. It is emphasized that, according to common practice, the various features of the drawing are not to scale. On the contrary, the dimensions of the various features are arbitrarily expanded or reduced for clarity. Included in the drawing are the following figures:
Fig. 1 is a perspective view of an exemplary embodiment of an infusion pump of the present invention;
Fig. 2 is a perspective view of an infusion pump without a keypad assembly attached thereto;
Fig. 3 is a perspective view of an exemplary keypad assembly according to an embodiment of the present invention; and
Fig. 4 is a perspective view of a frame for the keypad assembly shown in Figure 3.

### DETAILED DESCRIPTION OF THE INVENTION

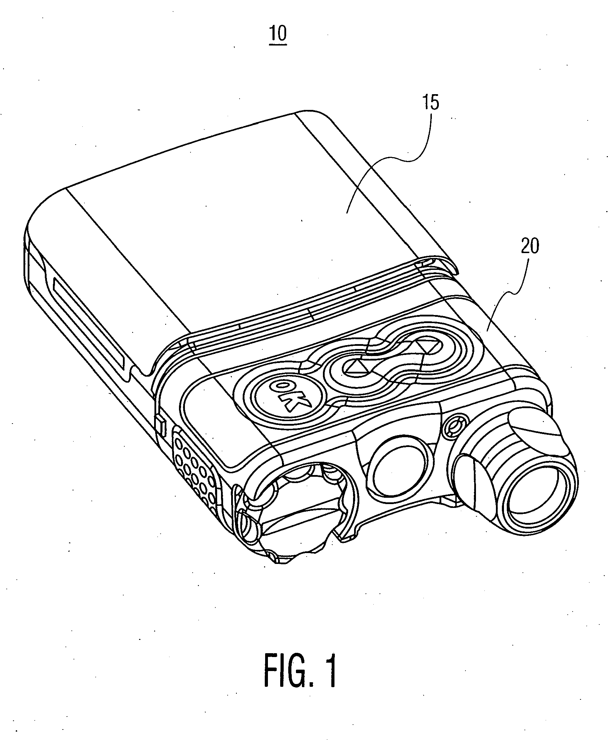

Referring now to the figures, Figure 1 is an illustration of an exemplary infusion pump according to an embodiment of the present invention. Infusion pump 10 includes keypad assembly 20 coupled to pump casing 15 forming a hermetic seal. As used herein "hermetic seal" or "hermetically sealed" means that the relationship between keypad assembly 20 and the pump casing 15 is made impervious to air and other fluids. The electronic parts (e.g., a flexible circuit disposed on a fexible circuit board) secured between keypad assembly 20 and the pump casing 15 are thus shielded against the entry of air and moisutre to maintain the safety and quality of circuitry on the flexible circuit, and the operation of the infusion pump.

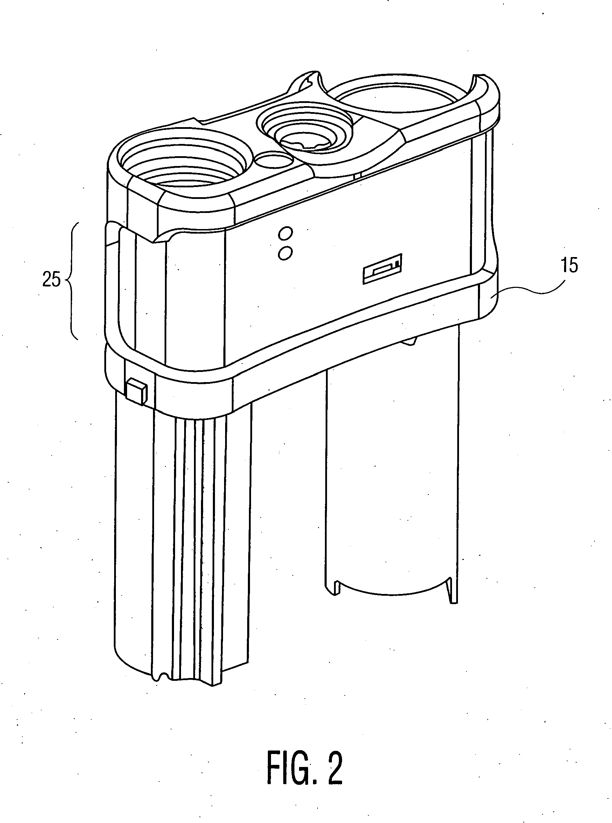

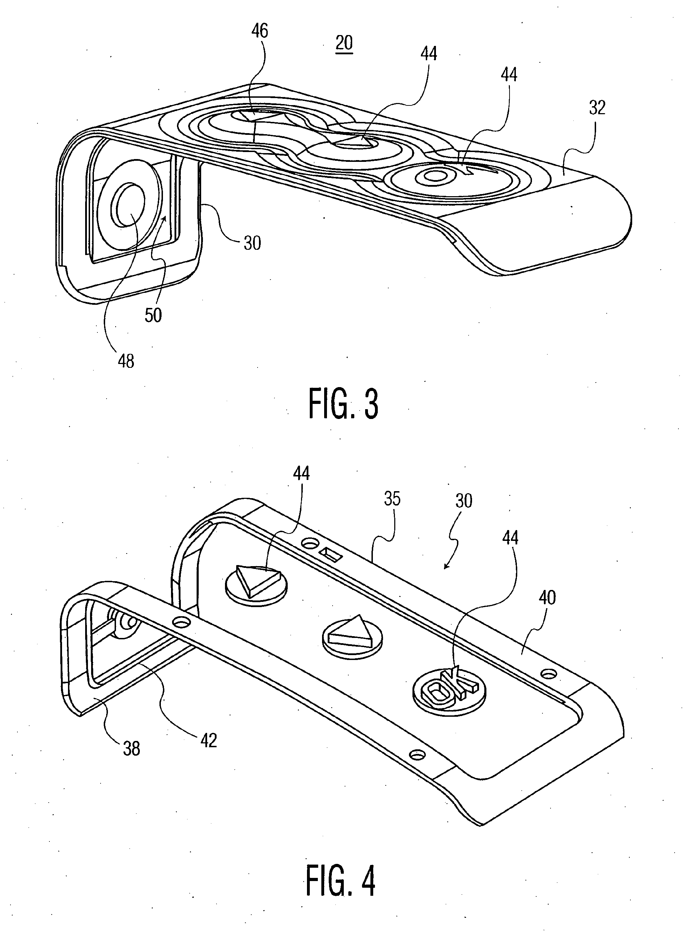

Figure 2 illustrates infusion pump 10 of Figure 1 with keypad assembly 20 removed from pump casing 15 to expose keypad assembly area 25. Keypad assembly area 25 is sized and shaped to receive keypad assembly 20 and a flexible circuit 50 (not shown in Fig. 3).

Keypad assembly 20 is shown in more detail in Figures 3 and 4. An exemplary keypad assembly 20 comprises a frame 30 and a keypad 32 overmolded to frame 30. Frame 30 defines a perimeter 35 sized to mate with keypad assembly area 25.

Keypad assembly area 25, pump casing 15, and frame 30 are made from a thermoplastic material suitable for medical applications. Typical materials include, but are not limited to, acrylonitrile butadiene styrene (ABS), acrylic, ethylene-vinyl acetate (EVA), ethylene vinyl alcohol (EVAL), liquid crystal polymer (LCP), polyacetal (POM or Acetal), polyacrylates (Acrylic), polyacrylonitrile (PAN or Acrylonitrile), polyamide (PA or Nylon) polyaryletherketone (PAEK or Ketone), polybutadiene (PBD), polybutylene (PB), polybutylene terephthalate (PBT), polyethylene terephthalate (PET), polycyclohexylene dimethylene terephthalate (PCT), polycarbonate (PC), polyhydroxyalkanoates (PHAs), polyketone (PK), polyester, polyethylene (PE), polyetheretherketone (PEEK), polyetherimide (PEI), olyethersulfone (PES), polyethylenechlorinates (PEC), polyimide (PI), polylactic acid (PLA), polymethylpentene (PMP), polyphenylene oxide (PPO), polyphenylene sulfide (PPS), polyphthalamide (PPA), polypropylene (PP), polystyrene (PS), polysulfone (PSU), polyvinyl chloride (PVC), TPU, thermoplastic elastomers (TPE), spectralon, and mixtures of any two or more thereof.

According to an exemplary embodiment, the material comprising frame 30 and the material comprising pump casing 15 and keypad assembly area 25 are not necessarily the same thermoplastic materials. If different, the material comprising frame 30 and the material comprising pump casing 15 and keypad assembly area 25 must be capable of forming a hermetic seal there between through the use of a suitable adhesive. Preferably, the material comprising frame 30 and the material comprising pump casing 15 and keypad assembly area 25 are the same thermoplastic material. More preferably, the material comprising frame 30, pump casing 15, and keypad assembly area 25 is polycarbonate.

Frame 30 has an adhesive surface 38. On adhesive surface 38 side of frame 30 is disposed a layer of adhesive. This adhesive chemically bonds adhesive surface 38 of frame 30 to keypad assembly area 25 of pump casing 15. Suitable adhesives include acrylics, epoxies, room temperature vulcanization chemicals, and mixtures of any two or more thereof. To maintain the adhesive layer on adhesive surface 38, frame 30 includes an adhesive stop edge 42.

Frame 30 also has an overmold surface 40. On overmold surface 40 side of frame 30 is molded keypad 32. During the molding process, for example an overmolding process, the thermoplastic overmolded keypad forms an integral bond to overmold surface 40 side of frame 30. To ensure that a proper hermetic seal has formed over the entirety of frame 30, the overmold cavity in the keypad mold must be completely filled during the injection molding process. High temperatures and pressures are necessary to ensure a complete seal.

An exemplary method of forming the integral bond and the resulting hermetic seal is to form frame 30, for example using poured, die cast, or injection molding techniques. Once frame 30 is formed, frame 30 is inserted into the keypad mold (not shown) over which molten material forming keypad 32 is injected. The high temperature of the molten keypad material forms a hermetic seal with the formed frame 30.

Keypad 32 is made from a suitable flexible elastomeric material. One such exemplary material includes silicones, defined generally as inorganic-organic polymers with the chemical formula [R₂SiO]n, where R = organic groups such as methyl, ethyl, and phenyl. Other exemplary materials include natural rubbers (NR) or synthetic rubbers, such as polyisoprenes (IR) including butyl rubber (copolymer of isobutylene and isoprene, IIR) and halogenated butyl rubbers (chloro butyl rubber: CIIR; bromo butyl rubber: BIIR), polybutadienes (BR) including styrene-butadiene rubber (copolymer of polystyrene and polybutadiene, SBR), nitrile rubber (copolymer of polybutadiene and acrylonitrile, NBR), and hydrated Nitrile Rubbers (HNBR); polychloroprene, ethylene propylene rubber (EPM), ethylene propylene diene rubber (EPDM), epichlorohydrin rubber (ECO), polyacrylic rubber (ACM, ABR), silicone rubber (SI, Q, VMQ), fluorosilicone rubber (FVMQ), fluoroelastomers (FKM, FPM), perfluoroelastomers (FFKM), tetrafluoro ethylene/propylene rubbers (FEPM), chlorosulfonated polyethylene (CSM), and ethylene-vinyl acetate (EVA).

According to an exemplary embodiment, the material comprising keypad 32 and the material comprising frame 30 may be different materials. Preferably, the material comprising keypad 32 is TPU and the material comprising pump casing 15 is polycarbonate.

Illustrated in Figures 3 and 4 are function keys 44. According to an exemplary embodiment, keypad 32 has four function keys. Function keys 44 can be configured into any desired shape or size depending on the function associated with the key. As illustrated in the non-limiting example of Figure 3, there is one function key that has the form of the term "OK." The function key may be made from a thermoplastic or elastomeric material, and may be the same material that comprises frame 30 or the same material the comprises keypad 32. Function keys 44 may be integrated within the shape of keypad 32 or formed from another material confined by keypad 32, as is shown in Figure 3.

According to one exemplary embodiment, function keys 44 are made from the same material as frame 30 and are confined by keypad 32 formed during the overmold process. In this embodiment, function keys 44 have an external surface 46 and an internal surface 48. Function keys 44 are positioned such that internal surface 48 corresponds to a contact point on a flexible circuit 50 disposed on the internal side of the keypad, which contact point controls a function of the infusion pump. Flexible circuit (Not Shown) may be disposed on a substrate such as a flexible circuit board in area 50. The function keys can be sized and/or shaped to correspond with an operation of the infusion pump allowing the user to tactically identify the function key and thus control the operation of the infusion pump.

An exemplary method of manufacturing the infusion pump of the present invention includes molding a pump casing by any means known to one of ordinary skill in the art. The frame of the keypad assembly is also molded by any means known to one of ordinary skill in the art. Preferably, the frame is formed by injection molding. Once the frame is formed, the frame is placed into the keypad mold, over which molten material forming the keypad is injected as would be understood to one of ordinary skill in the art. The molten material of the keypad fuses or bonds with the overmold surface of the frame. After the keypad assembly is formed, a bead or layer of adhesive is placed onto the adhesive surface of the frame within the keypad assembly. Optionally, the adhesive surface of the frame may be cleaned with a solvent to remove residues from previous formation/handling process steps. A thin flexible circuit is placed in electrical connection with the electric components of the pump via an input within the keypad assembly area. The keypad assembly is then placed over the flexible circuit. The function keys are aligned with the electrical contacts of the flexible circuit, and the frame of the keypad assembly is chemically bonded to the keypad assembly area. The fusion of the keypad material to the frame during the overmolding process and the chemical bond of the frame to the keypad assembly area hermetically seals the flexible circuit within the pump casing.

### Drop Test

According to an exemplary embodiment of the present invention, the hermetic seal formed between the keypad assembly and the keypad assembly area of the infusion pump has a seal strength sufficient to withstand a Drop Test from 1 meter onto a concrete surface.

The following test procedure verifies the functionality and enclosure integrity of the infusion pump after it is subjected to the Drop Test. This Drop Test is performed in accordance with BS EN 60601-1 and IEC 60601-2-24 guidelines, which are incorporated herein by reference.

The infusion pump is positioned to a specific attitude such that when dropped, it impacts the floor on a first corner, a second corner, or flat portion of the pump. The three starting attitudes were selected to represent most likely drop situations in field (i.e., if pump were dropped while infusion set was attached to user, infusion set would most likely re-direct pump so that one of two top corners of pump would contact ground first). The Drop Test procedure is as follows:
1. An activated pump is held in one of the three above-described attitudes such that the lowest point of the pump is maintained 1 meter above a concrete surface;
2. The pump is released and allowed to fall (under gravity) onto the concrete surface;
3. The pump is visually inspected for gross damage after the drop and is allowed to make one delivery (approximately 3 minutes) to see if pump alarms;
4. A leak test is performed on the pump (see Leak Test below);
5. The pump is then held in a second attitude as described-above and steps 2-4 are repeated;
6. The pump is then held in the final attitude as described-above and steps 2-4 are repeated.

After the pump is dropped from each starting attitude, the pump is visually inspected for damage, noting particularly, separation of the keypad or keypad assembly from the pump. A series of function and operational tests are also performed, including verifying that the function keys perform properly. If the pump functions properly and is structurally sound, the pump passes the Drop Test.

### Leak Test

According to an exemplary embodiment of the present invention, the hermetic seal formed between the keypad assembly to the keypad assembly area of the infusion pump remains sealed upon continuous immersion in about 12.7 ft of water for 24 hours.

The following test verifies that the integrity of the hermetic seal formed between the keypad assembly to the keypad assembly area of the infusion pump, particularly, after the pump is subjected to the Drop Test as defined above. This test verifies the electronic components of the pump are sufficiently protected against effects of continuous immersion in water. Specifically, this test verifies that there is no ingress of water in quantities causing harmful effects to operation of the pump when the pump is continuously immersed in 12.7 feet of water for 24 hours. The Leak Test procedure is as follows:
1. The pump is placed inside a pressure chamber filled with a sufficient quality of room temperature water such that the pump surface is at least 1 inch below the surface of the water; and
2. The pressure in the pressure chamber is increased to 5.75 (+0.25/-0.00) psi and maintained at this pressure for 24 hours.

After the 24 hour period, the pressure in the chamber is reduced to atmospheric pressure and a series of visual inspections and function checks are performed on the pump. These checks include:
a. Visually inspecting the keypad and keypad assembly area for signs of water ingress;
b. Confirming an audible alarm, if any;
c. Verifying that the pump responds to the function key push and the corresponding operation of the function keys perform properly (e.g., the pump activates when an ENTER function key is depressed);
d. Verifying that the LCD backlight display screen of the pump illuminates when a function key is pressed; and
e. Verifying operational accuracy of the pump by performing a pump cycle.

If the pump functions properly and is structurally sound, the pump passes the Leak Test.

Although the invention is illustrated and described herein with reference to specific embodiments, the invention is not intended to be limited to the details shown. Rather, various modifications may be made in the details within the scope and range of equivalents of the claims and without departing from the invention.

## Claims

1. An infusion pump comprising:
a pump casing comprising a first material and defining a keypad assembly area; and
a keypad assembly forming a hermetic seal over the keypad assembly area, the keypad assembly comprising:
(i) a frame comprising a second material and defining a perimeter sized to mate with the keypad assembly area, the frame having an adhesive surface and an overmold surface, and
(ii) a keypad comprising a third material and in direct contact with the overmold surface of the frame.

2. The infusion pump of claim 1, wherein the keypad assembly further comprises function keys.

3. The infusion pump of claim 2, wherein the keypad defines openings adapted to receive the function keys, and wherein each function key corresponds to an operational function of the infusion pump.

4. The infusion pump of claim 3, wherein the keypad defines four function keys.

5. The infusion pump of claim 1, wherein the adhesive surface of the frame defines an adhesive stop.

6. The infusion pump of claim 1, wherein the first material and the second material are the same.

7. The infusion pump of claim 6, wherein the second material is different than the third material.

8. The infusion pump of claim 1, wherein the first and second material are different from the third material.

9. The infusion pump of claim 1, wherein the first material and the second material are a thermoplastic material selected from the group consisting of acrylonitrile butadiene styrene (ABS), acrylic, ethylene-vinyl acetate (EVA), ethylene vinyl alcohol (EVAL), liquid crystal polymer (LCP), polyacetal (POM or Acetal), polyacrylates (Acrylic), polyacrylonitrile (PAN or Acrylonitrile), polyamide (PA or Nylon) polyaryletherketone (PAEK or Ketone), polybutadiene (PBD), polybutylene (PB), polybutylene terephthalate (PBT), polyethylene terephthalate (PET), polycyclohexylene dimethylene terephthalate (PCT), polycarbonate (PC), polyhydroxyalkanoates (PHAs), polyketone (PK), polyester, polyethylene (PE), polyetheretherketone (PEEK), polyetherimide (PEI), olyethersulfone (PES), polyethylenechlorinates (PEC), polyimide (PI), polylactic acid (PLA), polymethylpentene (PMP), polyphenylene oxide (PPO), polyphenylene sulfide (PPS), polyphthalamide (PPA), polypropylene (PP), polystyrene (PS), polysulfone (PSU), polyvinyl chloride (PVC), thermoplastic polyurethane elastomers (TPU), thermoplastic elastomers (TPE), spectralon, and mixtures of any two or more thereof.

10. The infusion pump of claim 9, wherein the first material and the second material are polycarbonate.

11. The infusion pump of claim 1, wherein the third material is a material selected from the group consisting of silicones, natural rubbers, synthetic rubbers, and mixtures of any two or more thereof.

12. The infusion pump of claim 11, wherein the third material is a silicone.

13. The infusion pump of claim 1, wherein the adhesive surface of the frame is chemically adhered to the keypad assembly area of the infusion pump casing with an adhesive material selected from the group consisting of acrylics, epoxies, room temperature vulcanization chemicals, and mixtures thereof.

14. The infusion pump of claim 1, wherein the infusion pump remains sealed upon continuous immersion in about 12.7 ft of water for 24 hours.

15. The infusion pump of claim 1, wherein the hermetic seal between the keypad assembly and the keypad assembly area of the infusion pump is of a sufficient seal strength to withstand a drop test from 1 meter onto a concrete surface.

16. A method of manufacturing an infusion pump comprising:
providing a pump casing comprising a first material and having a keypad assembly area;
molding a frame comprising a second material and defining a perimeter sized to mate with the keypad assembly area, the frame having an adhesive surface and an overmold surface;
overmolding a keypad onto the frame; and
adhering the adhesive surface of the keypad frame to the keypad assembly area of the pump casing with an adhesive to provide a hermetic seal to protect components within the pump casing.

17. The method of claim 16, wherein the overmolding step comprises:
inserting the molded frame into a keypad mold, and
applying a molten third material into the keypad mold containing the molded frame to bond the keypad to the overmold surface of the frame forming a keypad assembly.

18. A method of assembling an infusion pump having a keypad assembly hermetically sealed to a keypad assembly area of a pump casing of the infusion pump, the keypad assembly comprising (i) a frame defining a perimeter sized to mate with the keypad assembly area, the frame having an adhesive surface and an overmold surface, and (ii) a keypad in direct contact with the overmold surface of the frame and having function keys, the method of assembly comprising:
cleaning the keypad assembly area with a solvent;
placing adhesive on the adhesive surface of the frame of the keypad assembly;
aligning the adhesive surface of the frame perimeter with the keypad assembly area of the infusion pump casing; and
hermetically sealing the keypad assembly to the keypad assembly area of the infusion pump casing.
